(19) Europäisches Patentamt
European Patent Office
Office européen des brevets

(11) Veröffentlichungsnummer: **0 575 782 A1**

(12) **EUROPÄISCHE PATENTANMELDUNG**

(21) Anmeldenummer: **93108736.5**

(51) Int. Cl.5: **C07D 271/08**, A61K 31/41

(22) Anmeldetag: **01.06.93**

(30) Priorität: **20.06.92 DE 4220264**

(43) Veröffentlichungstag der Anmeldung:
**29.12.93 Patentblatt 93/52**

(84) Benannte Vertragsstaaten:
**AT BE CH DE DK ES FR GB GR IE IT LI NL PT SE**

(71) Anmelder: **CASSELLA Aktiengesellschaft**
**Hanauer Landstrasse 526**
**D-60386 Frankturt(DE)**

(72) Erfinder: **Schönafinger, Karl, Dr.**
**Holunderweg 8**
**W-8755 Alzenau(DE)**
Erfinder: **Bohn, Helmut, Dr.**
**Am Kranzbergring 11**
**W-6369 Schöneck 1(DE)**

(74) Vertreter: **Urbach, Hans-Georg, Dr. et al**
**Hanauer Landstrasse 526**
**D-60386 Frankturt (DE)**

(54) **Phenyl-1,2,5-oxadiazol-carbonamid-2-oxide zur Behandlung von cardiovasculären Störungen, insbesondere von Elektionstörungen.**

(57) Die vorliegende Erfindung betrifft Phenyl-1,2,5-oxadiazol-carbonamid-2-oxide der allgemeinen Formel I

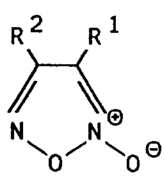

(I)

worin einer der Reste $R^1$ und $R^2$ für

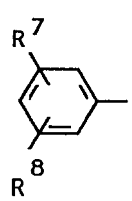

und der andere für

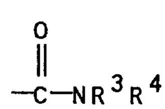

steht, und $R^3$ und $R^4$ wie in Anspruch 1 angegeben definiert sind, Verfahren zu ihrer Herstellung und ihre Verwendung.

EP 0 575 782 A1

Die vorliegende Erfindung betrifft Phenyl-1,2,5-oxadiazol-carbonamid-2-oxide, Verfahren zu ihrer Herstellung und ihre Verwendung.

4-Phenyl-1,2,5-oxadiazol-3-carbonsäureamid sowie 3-Phenyl-1,2,5-oxadiazol-4-carbonsäureamid sind bereits bekannt und in Liebigs Ann. Chem. 1990, 335 - 338 beschrieben. Ebenso sind die entsprechenden Phenyl- und Hexylamide bekannt und in Ann. Chim. (Rome) 58 (1968), 200 - 212 beschrieben. Über die pharmakologischen Eigenschaften dieser Substanzklasse ist allerdings noch nichts bekannt.

Die vorliegende Erfindung betrifft Phenyl-1,2,5-oxadiazol-carbonamid-2-oxide der allgemeinen Formel I

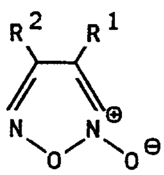

(I)

worin einer der Reste $R^1$ und $R^2$ für

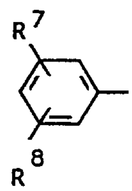

und der andere für

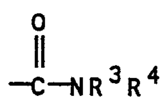

steht,
wobei
$R^3$ und $R^4$ unabhängig voneinander $(C_1-C_5)$-Alkyl, $(C_5-C_7)$--Cycloalkyl, $-(CH_2)_n-NR^5R^6$, $-(CH_2)_n-OR^5$, $-(CH_2)_m-COOR^5$, $-CH(Alk)-COOR^5$, $-(CH_2)_m-CONR^5R^6$, $-CH(Alk)-CONR^5R^6$,

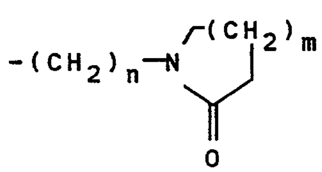

,

$-(CH_2)_m$-Aryl oder $-(CH_2)_m$-Heteroaryl und $R^4$ auch Wasserstoff bedeuten oder $R^3$ und $R^4$ zusammen mit dem diese bindenden Stickstoffatom einen Heterocyclus bilden;
$R^5$ und $R^6$ unabhängig voneinander Wasserstoff, $(C_1-C_6)$-Alkyl, $(C_5-C_7)$-Cycloalkyl, Benzyl, Phenethyl bedeutet; Alk $(C_1-C_6)$-Alkyl bedeutet;
n für 2, 3 oder 4 und
m für 1, 2 oder 3 stehen; und
$R^7$ und $R^8$ unabhängig voneinander Wasserstoff, $(C_1-C_4)$-Alkyl, $(C_1-C_4)$-Alkoxy, Fluor, Chlor, Brom, Nitro oder Trifluormethyl bedeuten;
sowie deren pharmakologisch annehmbare Säureadditionsverbindungen.

Die für $R^3$, $R^4$, $R^5$, $R^6$, $R^7$, $R^8$ oder Alk stehenden ($C_1$-$C_6$)-, ($C_1$-$C_5$)- bzw. ($C_1$-$C_4$)-Alkylgruppen können geradkettig oder verzweigt sein. Beispiele sind Methyl, Ethyl, n-Propyl, i-Propyl, n-Butyl, i-Butyl, sek.-Butyl, tert.-Butyl oder Hexyl. Entsprechendes gilt für $R^7$ oder $R^8$ stehende ($C_1$-$C_4$)-Alkoxygruppen.

Für $R^3$, $R^4$, $R^5$ oder $R^6$ stehendes ($C_5$-$C_7$)-Cycloalkyl bedeutet bevorzugt Cyclopentyl und Cyclohexyl.

In der für $R^3$ oder $R^4$ stehenden $-(CH_2)_m$-Aryl-Gruppe ist Aryl bevorzugt 6- bis 14-gliedrig. Bevorzugte Reste $-(CH_2)_m$-Aryl sind Phenyl, Benzyl und Phenylethyl.

In der für $R^3$ oder $R^4$ stehenden $-(CH_2)_m$-Heteroaryl-Gruppe ist Heteroaryl bevorzugt 5- bis 7-gliedrig und leitet sich beispielsweise von Pyrrol, Pyrrolidin, Imidazol, Pyridin, Piperidin, Morpholin oder Piperazin ab.

Die Aryl- und Heteroarylgruppen können gegebenenfalls auch ein- oder mehrfach substituiert sein. Geeignete Substituenten sind beispielsweise ($C_1$-$C_4$)-Alkyl, ($C_1$-$C_4$)-Alkoxy, Amino, ($C_1$-$C_4$)-Alkylamino, Di-($C_1$-$C_4$)-alkylamino, ($C_1$-$C_6$)-Alkanoylamino, Halogen, vorzugsweise Fluor, Chlor oder Brom, Hydroxy, Nitro oder Cyano.

Ein aus $R^3$, $R^4$ und dem diese bindenden Stickstoffatom gebildeter Heterocyclus ist beispielsweise Pyrrolidin, Piperidin, Morpholin oder Piperazin, wobei am Piperazin das zweite Stickstoffatom auch durch einen Rest $R^5$ substituiert sein kann.

Die Reste $R^7$ und $R^8$ können an alle freien Positionen des Phenylrings gebunden sein. Bevorzugt ist eine 2,3- oder 2,4-Substitution.

Bevorzugt bedeuten $R^3$ ($C_1$-$C_5$)-Alkyl und $R^4$ Wasserstoff. Es ist darüberhinaus bevorzugt, wenn $R^3$ $-(CH_2)_nN((C_1$-$C_6)$-Alkyl$)_2$ und $R^4$ Wasserstoff bedeuten. Das für $R^1$ oder $R^2$ stehende

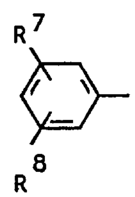

bedeutet bevorzugt Phenyl.

Ganz besonders bevorzugt bedeuten $R^3$ $-(CH_2)_nN((C_1$-$C_6)$-Alkyl$)_2$ und $R^4$, $R^7$ und $R^8$ Wasserstoff.

Die Verbindungen der allgemeinen Formel I können beispielsweise dadurch hergestellt werden, daß eine Verbindung der allgemeinen Formel II

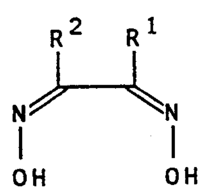

(II)

worin $R^1$ und $R^2$ wie oben angegeben definiert sind, oxidiert wird.

Als Oxidationsmittel können dabei herkömmliche Reagentien wie zum Beispiel Halogene, Alkalihypochlorite, Blei(IV)-acetat, Eisen-III-salze wie beispielsweise rotes Blutlaugensalz oder nitrose Gase, wie beispielsweise $N_2O_4$ eingesetzt werden. Die Umsetzung wird bevorzugt in einem Lösungsmittel, wie beispielsweise Wasser, einem Alkohol, einem Ether, Essigester, Methylenchlorid, Cyclohexan, DMF, DMSO, Benzol, Toluol oder Chlorbenzol bei Temperaturen von -10°C bis 50°C, vorzugsweise von -5°C bis 25°Causgeführt.

Bei der genannten Oxidation fallen in der Regel die Verbindungen der allgemeinen Formel I in Form von Isomerengemischen an. Diese lassen sich aber durch bekannte Methoden wie Umkristallisieren oder chromatographische Methoden, insbesondere Säulenchromatographie, trennen.

Isomerengemische werden auch erhalten, wenn ein reines Isomeres in Substanz oder in einem inerten Lösungsmittel gelöst auf Temperaturen von 50 bis 200°C erhitzt oder bei 0 bis 50°C photolysiert wird. Durch Trennung des so erhaltenen Gemisches ist es somit möglich, ein Isomeres in das andere umzuwandeln.

Die Verbindungen der allgemeinen Formel II können hergestellt werden durch Umsetzung von Verbindungen der allgemeinen Formel III

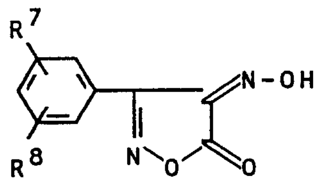

(III)

mit einem Amin $HNR^3R^4$, wobei $R^3$ und $R^4$ wie oben angegeben definiert sind. Die Umsetzung wird bevorzugt in einem inerten Lösungsmittel bei Temperaturen von 0 bis 50°C ausgeführt.

Die Herstellung der Verbindung der Formel III ist beschrieben in Ann.Chim.(Rome) (1968), 58(2), 189-199, und in Ann.Chim.(Rome) (1959), 49, 2083-2088.

Ein alternatives Verfahren zur Herstellung von erfindungsgemäßen Verbindungen der allgemeinen Formel I besteht darin, eine Verbindung der allgemeinen Formel IV

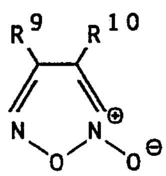

(IV)

worin einer der Reste $R^9$ und $R^{10}$ für

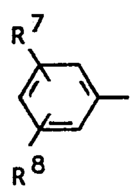

und der andere für eine reaktive Säuregruppe, wie zum Beispiel

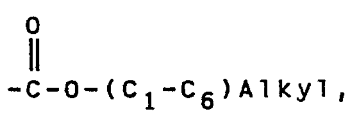 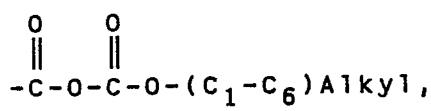

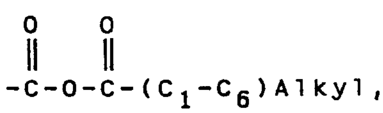 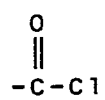 oder 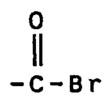 steht,

mit einem Amin $HNR^3R^4$, worin $R^3$ und $R^4$ wie oben angegeben definiert sind, umzusetzen.

Vorteilhafterweise wird die Reaktion in Gegenwart einer Base ausgeführt, die die entstehenden Säuren neutralisiert. Bevorzugte Basen sind Alkalicarbonate, wie Natrium- oder Kaliumhydrogencarbonat oder Natrium- oder Kaliumcarbonat, Alkalihydroxide, wie Natrium-, Kalium- oder Lithiumhydroxid, Alkalialkoholate, wie Natrium- oder Kaliummethylat, Natrium- oder Kaliumethylat oder Kaliumtert.-butylat, Alkalihydride, wie Natrium- oder Kaliumhydrid, Alkaliamide, wie Natriumamid oder Lithiumdiisopropylamid oder organische Basen wie Pyridin oder Triethylamin. Diese Basen werden bevorzugt in molaren Mengen eingesetzt. Als Lösungsmittel kommen beispielsweise Ether, THF, Alkohole, Toluol, DMF und DMSO in Frage. Die Temperaturen liegen bei 0 bis 100°C, bevorzugt 0 bis 50°C.

Gegebenenfalls können die nach einem der obenstehenden Verfahren hergestellten erfindungsgemäßen Verbindungen der allgemeinen Formel I durch Modifizierung der Substituenten in weitere erfindungsgemäße Verbindungen der allgemeinen Formel I umgewandelt werden.

Beispielsweise kann die Seitenkette $-CO-NH-C(CH_2)_mCOOR^5$ durch Umsetzung mit einem Amin $HNR^5R^6$ in die Seitenkette $-CO-NH-(CH_2)_mCONR^5R^6$ umgewandelt weren. Analoges ist mit der Seitenkette $-CO-NH-CH(Alk)-COOR^5$ möglich.

Nach den oben angegebenen Verfahren lassen sich beispielsweise erfindungsgemäße Verbindungen der allgemeinen Formel Ia

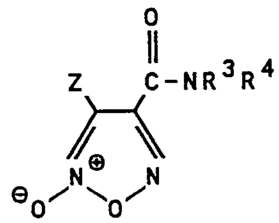

(Ia)

sowie der allgemeinen Formel Ib

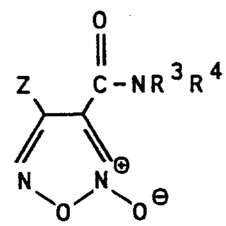

(Ib)

herstellen, worin Z jeweils für Phenyl, 2-, 3- oder 4-Methoxyphenyl oder für 3,4-Dimethoxyphenyl und $-NR^3R^4$ jeweils steht für:
$-NH_2$; $-NHCH_3$; $-NHCH_2CH_3$; $-NH(CH_2)_2CH_3$; $-NH(CH_2)_3CH_3$; $-NH(CH_2)_4CH_3$; $-NH(CH_2)_5CH_3$; $-NHcycloC_6H_{11}$; $-NHcycloC_5H_9$; $-NH(CH_2)_2N(CH_3)_2$; $-NH-(CH_2)_2N(CH_2CH_3)_2$; $-NH(CH_2)_2N-(CH_2CH_2CH_2CH_3)_2$; $-NH-(CH_2)_2N(CH(CH_3)_2)_2$; $-NH(CH_2)_2NHCH(CH_3)_2$; $-NH(CH_2)_2NHcycloC_6H_{11}$; $-NH-(CH_2)_3N(CH_2CH_3)_2$;

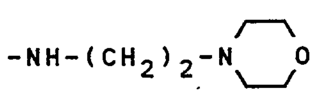; 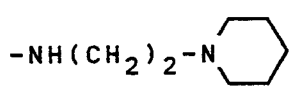; 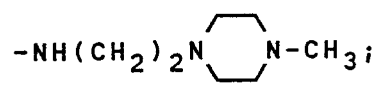;

$-NHCH_2CH(CH_3)_2$; $-NH(CH_2)_3N(CH_3)_2$;

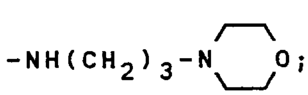; 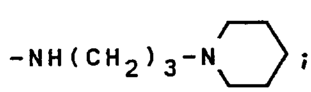; $-NHCH_2C(CH_3)_3$;

$-NH(CH_2)_4N(CH_3)_2$; $-NH(CH_2)_3NHcycloC_6H_{11}$; $-NH(CH_2)_3NHC(CH_3)_3$; $-NH(CH_2)_2OCH_3$; $-NH(CH_2)-_2OCH_2CH_3$; $-NH(CH_2)_2O(OH_2)CH_3$; $-NH(CH_2)_2OCH(CH_3)_2$; $-NH(CH_2)_3OCH_3$; $-NH(CH_2)_4OCH_3$; $-NH(CH_2)-_3OCH_2CH_3$; $-NH(CH_2)_3O(CH_2)_3CH_3$; $-NH(CH_2)_2OH$; $-NH(CH_2)_3OH$; $-NH(CH_2)_2OcycloC_5H_9$; $-NH(CH_2)-_2$Phenyl; $-NHCH_2$Phenyl; $-NH(CH_2)_3$Phenyl; $-NH(CH_2)_2$(3,4-Di-$OCH_3$-Phenyl); $-NHCH_2$(4-$OCH_3$-Phenyl); -$NHCH_2COOCH_3$; $-NHCH_2COOCH$; $-NHCH_2COOCH_2CH_3$; $-NHCH_2CONH_2$; $-NHCH_2CONHCH_3$; $-NHCH_2CON(CH_2CH_3)_2$; $-NH(CH_2)_2COOH$; $-NH(CH_2)_2COOCH(CH_3)_2$: $-NH(CH_2)_2CONHCH_2CH_3$; $-NH(CH_2)-_3COOH$; $-NH(CH_2)_3CONH(CH_2)_3CH_3$; $-NH(CH_2)_3CONH_2$; $-NH(CH_2)_3COOCH_2CH_3$; $-NHCH_2$(3-Pyridyl); -NH-$(CH_2)_2$(4-Pyridyl); $-NH(CH_2)_2$(4-Imidazolyl); $-NHCH_2$(2-Pyridyl); $-NHCH(CH_3)COOH$; $-NHCH(CH(CH_3)_2)-CONH_2$; $-NHCH(CH_2CH(CH_3)_2)CON(CH_3)_2$; $-NH(CH_2)_2$(2-Oxo-pyrrolidin-1-yl) oder $-NH(CH_2)_3$(2-Oxo-pyrrolidin-1-yl).

Erfindungsgemäße Verbindungen der allgemeinen Formel I, die eine basische Gruppe enthalten, können mit anorganischen oder organischen Sären Salze bilden. Geeignete Säuren für die Bildung

pharmakologisch annehmbarer Säureadditionssalze sind beispielsweise: Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Maleın-, Apfel-, Sulfamin-, Phenylpropion-, Glucon-, Ascorbin-, Isonicotin-, Methansulfon-, p-Toluolsulfon-, Zitronen- oder Adipinsäure. Die Säureadditionssalze können wie üblich durch Vereinigung der Komponenten, zweckmäßigerweise in einem geeigneten Lösungs- oder Verdünnungsmittel, hergestellt werden.

Die Verbindungen der allgemeinen Formel I und ihre pharmakologisch annehmbaren Säureadditionssalze besitzen wertvolle pharmakologische Eigenschaften. Am Modell der Kalium-depolarisierten Pulmonalarterie des Meerschweinchen führen sie in niedrigen Konzentrationen zu einer lang anhaltenden Relaxation. Diese Wirkung kann mit Oxyhämoglobin inhibiert werden, was auf einen NO-mediierten Mechanismus deutet. Stickstoffmonoxid führt als Aktivator der Guanylatcyclase zu einer Erhöhung von cyclischem Guanosinmonophosphat, welches im glatten Muskel eine Relaxation und in den Blutplättchen antiadhäsive und antiaggregatorische Wirkungen verursacht. Stickstoffmonoxid ist außerdem auch entscheidend beteiligt bei Lernvorgängen, bei der Regulation der Nierenfunktion, bei der Immunabwehr, beim septischen Schock und bei erektilen Dysfunktionen. Die erfindungsgemäßen Verbindungen können somit bei den genannten Indikationen eingesetzt werden. Vor allem aber haben sich NO-Donoren zur Behandlung und Prophylaxe von angina pectoris bewährt.

Verbindungen gemäß allgemeiner Formel I, in denen $R^3$ und $R^4$ Wasserstoff oder $R^3$ Hexyl oder Phenyl und $R^4$ Wasserstoff bedeuten, sind zwar als solche, wie oben angegeben, bereits beschrieben, über deren pharmakologische Eigenschaften ist aber bisher nichts bekannt. Es wurde nun gefunden, daß diese Verbindungen die gleichen pharmakologischen Eigenschaften wie die Verbindungen der allgemeinen Formel I aufweisen. Sie können analog den oben angegebenen Verfahren hergestellt werden.

Die Verbindungen der allgemeinen Formel I

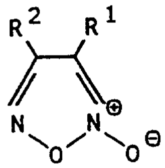

(I)

worin einer der Reste $R^1$ und $R^2$ für

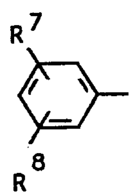

und der andere für

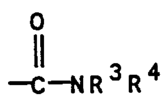

steht,

wobei

$R^3$ und $R^4$ unabhängig voneinander Wasserstoff, $(C_1-C_6)$-Alkyl, $(C_5-C_7)$Cycloalkyl, $-(CH_2)_n-NR^5R^6$, $-(CH_2)_n-OR^5$, $-(CH_2)_m-COOR^5$, $-CH(Alk)-COOR^5$, $-(CH_2)_m-CONR^5R^6$, $-CH(Alk)-CONR^5R^6$,

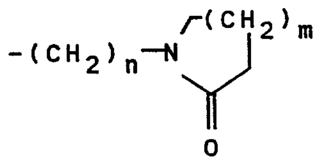

$-(CH_2)_m$-Aryl, $-(CH_2)_m$-Heteroaryl oder Phenyl bedeuten oder $R^3$ und $R^4$ zusammen mit dem diese bindenden Stickstoffatom einen Heterocyclus bilden;

$R^5$ und $R^6$ unabhängig voneinander Wasserstoff, ($C_1$-$C_6$)-Alkyl, ($C_5$-$C_7$)-Cycloalkyl, Benzyl, Phenethyl bedeuten;

Alk ($C_1$-$C_6$)-Alkyl bedeutet;

n für 2, 3 oder 4 und

m für 1, 2 oder 3 stehen; und $R^7$ und $R^8$ unabhängig voneinander Wasserstoff, ($C_1$-$C_4$)-Alkyl, ($C_1$-$C_4$)-Alkoxy, Fluor, Chlor, Brom, Nitro oder Trifluormethyl bedeuten und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Säureadditionssalzes davon, neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer pharmakologisch annehmbaren Säureadditionssalze und noch andere therapeutisch wirksame Stoffe enthalten.

Derartige andere therapeutisch wirksame Substanzen sind beispielsweise: $\beta$-Rezeptorenblocker, wie z.B. Propranolol, Pindolol, Metoprolol; Vasodilatatoren, wie z.B. Carbocromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Ramipril, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

Die Verbindungen der allgemeinen Formel I, ihre pharmakologisch annehmbaren Säureadditionsssalze und pharmazeutische Präparate, welche die Verbindungen der allgemeinen Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Darüberhinaus können sie auch zur Behandlung erektiler Dysfunktionen eingesetzt werden. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z. B. 2 bis 4 Teilverabreichungen aufgeteilt.

Beispiele

1. 4-Phenyl-1,2,5-oxadiazol-3-carbonsäuremethylamid-2-oxid und 3-Phenyl-1,2,5-oxadiazol-4-carbonsäuremethylamid-2-oxid

a) In eine Mischung von 9,5 g 3-Phenyl-4-hydroximinoisoxazol-5-on und 50 ml Methanol wird etwas mehr als die äquimolare Menge Methylamin (2 g) eingeleitet. Die Mischung wird 48 Stunden bei Raumtemperatur und dann im Eisbad gerührt. Anschließend wird der Niederschlag abgesaugt, mit wenig Methanol gewaschen und getrocknet.

Ausbeute: 8,7 g 3-Phenyl-2,3-dihydroximino-propionsäuremethylamid

Fp.: 149°C (Zers.)

b) Zu einer eisgekühlten Lösung von 7,2 g der Verbindung gemäß a) in 23 ml 2n Natronlauge werden 21 ml einer 14 %igen Natriumhypochlorit-Lösung getropft. Nach 15 Minuten wird der Niederschlag abgesaugt, mit Wasser gewaschen, getrocknet und säulenchromatographisch (Kieselgel; Cyclohexan: Essigester = 8 : 2) aufgetrennt. Es resultieren 2,9 g 4-Phenyl-Verbindung mit einem Fp. von 117 - 9°C (Verbindung 1a) und 2,4 g der 3-Phenyl-Verbindung mit einem Fp. von 134 - 6°C (Verbindung 1b).

2. 4-Phenyl-1,2,5-oxadiazol-3-carbonsäure-(2-diethylaminoethyl)-amid-2-oxid und 3-Phenyl-1,2,5-oxadiazol-4-carbonsäure-(2-diethylaminoethyl)-amid-2-oxid

a) Analog der Angabe in Beispiel 1a) wird 3-Phenyl-2,3-dihydroximino-propionsäure-(2-diethylaminoethyl)amid hergestellt. Fp.: 157 - 8 (Zers.).

b) Zu einer eisgekühlten Lösung von 10,7 g der Verbindung gemäß a) in 35 ml 2n Natronlauge werden 25 ml einer 14 %igen Natriumhypochlorit-Lösung getropft. Nach 20 Minuten wird das ölige Produktgemisch mit Essigester extrahiert und nach dem Einengen im Vakuum säulenchromatographisch aufgetrennt (Kieselgel; Cyclohexan: Aceton = 7 : 3). Dabei werden 4,5 g der 4-Phenyl-Verbindung (Verbindung 2a) und 2,9 g der 3-Phenyl-Verbindung (Verbindung 2b), jeweils als Öl, erhalten.

Analog den Beispielen 1 und 2 können erhalten werden:

3. 3-Phenyl-1,2,5-oxadiazol-4-carbonsäure-(2-hydroxyethyl)-amid-2-oxid (Fp.: 120 - 121°C) (Verbindung 3b) und 4-Phenyl-1,2,5-oxadiazol-3-carbonsäure-(2-hydroxyethyl)-amid-2-oxid (Fp.: 104 - 106°C) (Verbindung 3a) aus 3-Phenyl-2,3-dihydroximino-propionsäure-(2-hydroxyethyl)amid (Fp.: 179 - 81°C (Zers.)).

4. 3-(3,4-Dimethoxyphenyl)-1,2,5-oxadiazol-4-carbonsäuremethylamid-2-oxid (Fp.: 156 - 8°C) (Verbindung 4b) und 4-(3,4-Dimethoxyphenyl)-1,2,5-oxadiazol-3-carbonsäuremethylamid-2-oxid (Fp.: 145 - 8°C) (Verbindung 4a) aus 3-(3,4-Dimethoxyphenyl)-2,3-dihydroximino-propionsäuremethylamid (Fp.: 136°C (Zers.)).

5. 3-Phenyl-1,2,5-oxadiazol-4-carbonsäurebutylamid-2-oxid (Öl) (Verbindung 5b) und 4-Phenyl-1,2,5-oxadiazol-3-carbonsäurebutylamid-2-oxid (Fp.: 76 - 8°C) (Verbindung 5a) aus 3-Phenyl-2,3-dihydroximino-propionsäurebutylamid (Öl).

6. 3-Phenyl-1,2,5-oxadiazol-4-carbonsäure-(3-diethylaminopropyl)-amid-2-oxid (Öl) (Verbindung 6b) und 4-Phenyl-1,2,5-oxadiazol-3-carbonsäure-(3-diethylaminopropyl)-amid-2-oxid (Öl) (Verbindung 6a) aus 3-Phenyl-2,3-dihydroximino-propionsäure-(3-diethylaminopropyl)amid (Fp.: 157°C (Zers.)).

Außerdem können die Verbindungen der folgenden Tabelle erhalten werden:

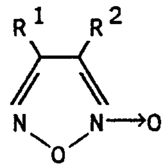

| | $R^1$ | $R^2$ | Fp. |
|---|---|---|---|
| 7a | 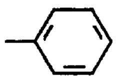 | 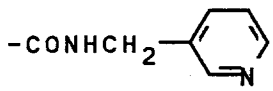 | 121-3°C |
| 7b | 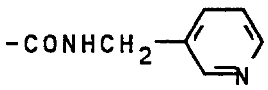 | 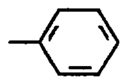 | 148-9°C |

| | $R^1$ | $R^2$ | Fp. |
| --- | --- | --- | --- |
| 8a | 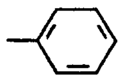 | -CONH-$(CH_2)_3$-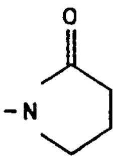 | 109-10°C |
| 8b | -CONH$(CH_2)_3$-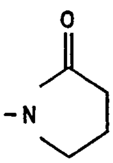 | 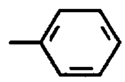 | 94-7°C |
| 9a | 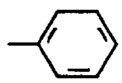 | -CONH-$(CH_2)_3$-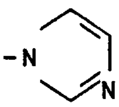 | 111-3°C |
| 9b | -CONH-$(CH_2)_3$-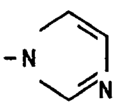 | 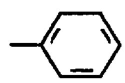 | 156-8°C |
| 10a | 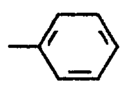 | -CONHCH$_2$-CO-NH$_2$ | 213-4°C |
| 10b | -CONHCH$_2$-CO-NH$_2$ | 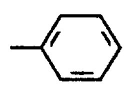 | 204-6°C |
| 11a | 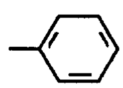 | -CO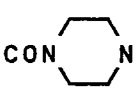-$CH_3$ | 106-8°C |
| 11b | -CO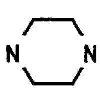-$CH_3$ | 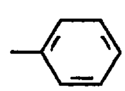 | 123-5°C |

| | $R^1$ | $R^2$ | Fp. |
|---|---|---|---|
| 12a | 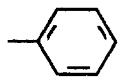 | $-CONH(CH_2)_2N(iPr)_2 \cdot HCl$ | 128-30°C |
| 12b | $-CONH(CH_2)_2N(iPr)_2 \cdot HCl$ | 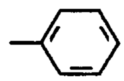 | ab 82°C Zers. |
| 13a | 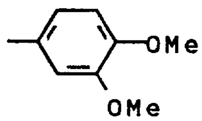 | $-CONH(CH_2)_2N(iPr)_2$ | 125-6°C |
| 13b | $-CONH(CH_2)_2N(iPr)_2$ | 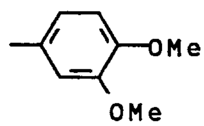 | 132-5°C |
| 14a | 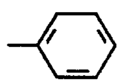 | $-CONH-CH_2$ 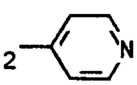 | 115-6°C |
| 14b | $-CONH-CH_2$ 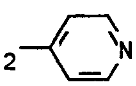 | 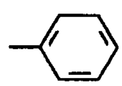 | 144-5°C |
| 15a | 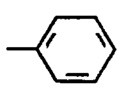 | $-CONH(CH_2)_2$ 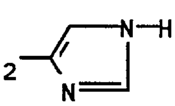 | 133-5°C |
| 15b | $-CONH(CH_2)_2$ 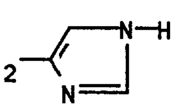 | 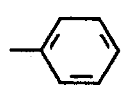 | 163-5°C |

| | $R^1$ | $R^2$ | Fp. |
|---|---|---|---|
| 16a | 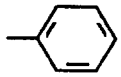 | $-CONH(CH_2)_2$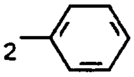 | 77-9°C |
| 16b | $-CONH(CH_2)_2$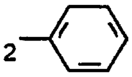 | 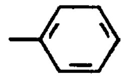 | 110-1°C |
| 17a | 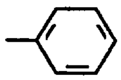 | $-CONH(CH_2)_2-NH_2$ | Öl |
| 17b | $-CONH(CH_2)_2-NH_2$ | 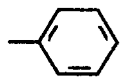 | Öl |
| 18a | 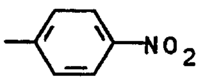 | $-CONH-(CH_2)_2-N(iPr)_2$ | 114-5°C |
| 18b | $-CONH(CH_2)_2-N(iPr)_2$ | 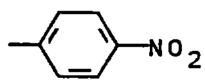 | 79-81°C |
| 19a | 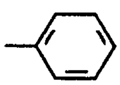 | $-CONH(CH_2)_3NMe_2$ | Öl |
| 19b | $-CONH(CH_2)_3NMe_2$ | 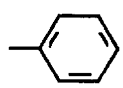 | 83-5°C |

20. 3-(3,4-Dimethoxiphenyl)-N-(pyrid-3-yl-methyl)-1,2,5-oxadiazol-4-carbonamid-2-oxid Fp.: 153°-155°C.

21. 4-(3,4-Dimethoxiphenyl)-N-(pyrid-3-yl-methyl)-1,2,5-oxadiazol-3-carbonamid-2-oxid Fp.: 111°-114°C.

22. 4-(4-Nitrophenyl)-N-(2-diisopropylamino-ethyl)-1,2,5-oxadiazol-3-carbonamid-2-oxid Fp.: 114°-115°C.

Die pharmakologische Wirkung der Verbindungen der allgemeinen Formel I wurde nach einer modifizierten Methode von Godfraind and Kaba (Arch.Int. Pharmacodyn. Ther. 196, (Suppl) 35 bis 49, 1972) und von Schüman et al (Naunyn-Schmiedeberg's Arch. Pharmacol. 289, 409 bis 418, 1975) ermittelt. Dabei werden Spiralstreifen der Arteria pulmonalis des Meerschweinchens nach Äquilibrierung in calciumfreier Tyrodelösung mit 40 mmol/l Kalium depolarisiert. Ein Zusatz von 0,5 mmol/l $CaCl_2$ löst dann eine Kontraktion aus.

Die relaxierende Wirkung der Prüfsubstanz wird durch kumulative Zugabe in 1/2 log 10 abgestuften Konzentrationen ermittelt. Aus der Konzentrationswirkungskurve (Abszisse: -log mol/l Prüfsubstanz, Ordinate: % Hemmung der maximalen Kontraktion, Mittelwert von 4 bis 6 Gefäßstreifen) wird die Konzentration der Prüfsubstanz ermittelt, welche die Kontraktion um 50 % hemmt (= $IC_{50}$, mol/l).

Folgende Werte wurden erhalten:

| Verbindung | $IC_{50}$ (mol/l) |
|---|---|
| 1a | $1 \cdot 10^{-6}$ |
| 4a | $5 \cdot 10^{-6}$ |
| 5a | $7 \cdot 10^{-7}$ |
| 6a | $4 \cdot 10^{-7}$ |
| 7a | $3 \cdot 10^{-7}$ |
| 7b | $2 \cdot 10^{-6}$ |
| 10a | $1 \cdot 10^{-6}$ |
| 12b | $2 \cdot 10^{-6}$ |
| 13a | $3 \cdot 10^{-7}$ |
| 14a | $5 \cdot 10^{-7}$ |
| 14b | $1 \cdot 10^{-6}$ |
| Molsidomin (Vergleich) | $3 \cdot 10^{-4}$ |
| Isosorbid-5-mononitrat (Vergleich) | $>1 \cdot 10^{-4}$ |

## Patentansprüche

1. Phenyl-1,2,5-oxadiazol-carbonamid-2-oxide der allgemeinen Formel I

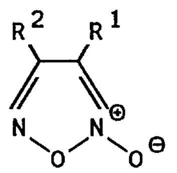

(I)

worin einer der Reste $R^1$ und $R^2$ für

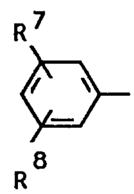

und der andere für

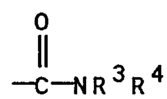

steht,
wobei
$R^3$ und $R^4$ unabhängig voneinander ($C_1$-$C_5$)-Alkyl, ($C_5$-$C_7$)--Cycloalkyl, -$(CH_2)_n$-$NR^5R^6$, -$(CH_2)_n$-$OR^5$, -$(CH_2)_m$-$COOR^5$, -CH(Alk)-$COOR^5$, -$(CH_2)_m$-$CONR^5R^6$, -CH(Alk)-$CONR^5R^6$,

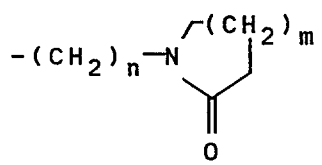,

-$(CH_2)_m$-Aryl oder -$(CH_2)_m$-Heteroaryl und $R^4$ auch Wasserstoff bedeuten oder $R^3$ und $R^4$ zusammen mit dem diese bindenden Stickstoffatom einen Heterocyclus bilden;
$R^5$ und $R^6$ unabhängig voneinander Wasserstoff, ($C_1$-$C_6$)-Alkyl, ($C_5$-$C_7$)-Cycloalkyl, Benzyl, Phenethyl bedeutet;
Alk ($C_1$-$C_6$)-Alkyl bedeutet;
n für 2, 3 oder 4 und
m für 1, 2 oder 3 stehen; und
$R^7$ und $R^8$ unabhängig voneinander Wasserstoff, ($C_1$-$C_4$)-Alkyl, ($C_1$-$C_4$)-Alkoxy, Fluor, Chlor, Brom, Nitro oder Trifluormethyl bedeuten;
sowie deren pharmakologisch annehmbare Säureadditionsverbindungen.

**2.** Phenyl-1,2,5-oxadiazol-carbonamid-2-oxide gemäß Anspruch 1, dadurch gekennzeichnet, daß $R^3$ ($C_1$-$C_5$)-Alkyl und $R^4$ Wasserstoff bedeuten.

**3.** Phenyl-1,2,5-oxadiazol-carbonamid-2-oxide gemäß Anspruch 1, dadurch gekennzeichnet, daß $R^3$ -$(CH_2)_nN((C_1$-$C_6)$-$Alkyl)_2$ und $R^4$ Wasserstoff bedeuten.

**4.** Phenyl-1,2,5-oxadiazol-carbonamid-2-oxide gemäß einem oder mehreren der Ansprüche 1 bis 3, dadurch gekennzeichnet, daß das für $R^1$ oder $R^2$ stehende

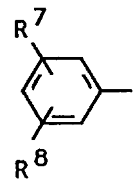

Phenyl ist.

**5.** Phenyl-1,2,5-oxadiazol-carbonamid-2-oxide gemäß einem oder mehreren der Ansprüche 1, 3 und 4, dadurch gekennzeichnet, daß $R^3$ $-(CH_2)_nN((C_1-C_6)\text{-Alkyl})_2$ und $R^4$, $R^7$ und $R^8$ Wasserstoff bedeuten.

**6.** Verfahren zur Herstellung von Phenyl-1,2,5-oxadiazol-carbonamid-2-oxiden der allgemeinen Formel I gemäß einem oder mehreren der Ansprüche 1 bis 5, dadurch gekennzeichnet, daß eine Verbindung der allgemeinen Formel II

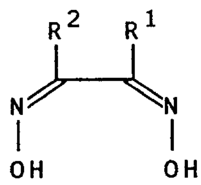

(II)

worin $R^1$ und $R^2$ wie in Anspruch 1 angegeben definiert sind, oxidiert wird.

**7.** Verfahren zur Herstellung von Phenyl-1,2,5-oxadiazol-carbonamid-2-oxiden der allgemeinen Formel I gemäß einem oder mehreren der Ansprüche 1 bis 5, dadurch gekennzeichnet, daß eine Verbindung der allgemeinen Formel IV

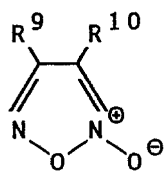

(IV)

worin einer der Reste $R^9$ und $R^{10}$ für

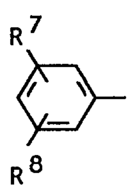

und der andere für eine reaktive Säuregruppe steht, mit einem Amin $HNR^3R^4$, worin $R^3$ und $R^4$ wie in Anspruch 1 angegeben definiert sind, umgesetzt wird.

**8.** Verwendung von Phenyl-1,2,5-oxadiazol-carbonamid-2-oxid der allgemeinen Formel I

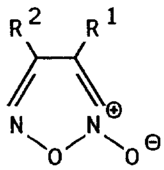

(I)

worin einer der Reste $R^1$ und $R^2$ für

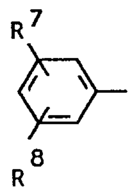

und der andere für

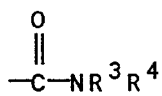

steht,
wobei
$R^3$ und $R^4$ unabhängig voneinander Wasserstoff, $(C_1-C_6)$-Alkyl, $(C_5-C_7)$Cycloalkyl, $-(CH_2)_n-NR^5R^6$, $-(CH_2)_n-OR^5$, $-(CH_2)_m-COOR^5$, -CH(Alk)-$COOR^5$, $-(CH_2)_m-CONR^5R^6$, -CH(Alk)-$CONR^5R^6$,

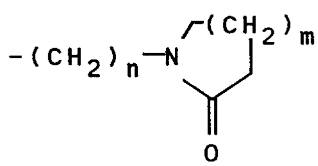

$-(CH_2)_m$-Aryl, $-(CH_2)_m$-Heteroaryl oder Phenyl bedeuten oder $R^3$ und $R^4$ zusammen mit dem diese bindenden Stickstoffatom einen Heterocyclus bilden;
$R^5$ und $R^6$ unabhängig voneinander Wasserstoff, $(C_1-C_6)$-Alkyl, $(C_5-C_7)$-Cycloalkyl, Benzyl, Phenethyl bedeutet;
Alk $(C_1-C_6)$-Alkyl bedeutet;
n für 2, 3 oder 4 und
m für 1, 2 oder 3 stehen; und
$R^7$ und $R^8$ unabhängig voneinander Wasserstoff, $(C_1-C_4)$-Alkyl, $(C_1-C_4)$-Alkoxy, Fluor, Chlor, Brom, Nitro oder Trifluormethyl bedeuten oder eines pharmakologisch annehmbaren Säureadditionssalzes davon zur Bekämpfung und Vorbeugung von Erkrankungen des kardiovaskulären Systems.

**9.** Verwendung von Phenyl-1,2,5-oxadiazol-carbonamid-2-oxid der allgemeinen Formel I

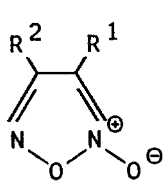

(I)

worin einer der Reste $R^1$ und $R^2$ für

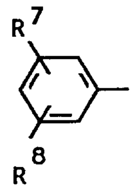

und der andere für

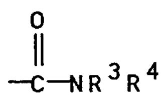

steht,
wobei
$R^3$ und $R^4$ unabhängig voneinander Wasserstoff, $(C_1\text{-}C_6)$-Alkyl, $(C_5\text{-}C_7)$Cycloalkyl, $-(CH_2)_n-NR^5R^6$, $-(CH_2)_n-OR^5$, $-(CH_2)_m-COOR^5$, $-CH(Alk)-COOR^5$, $-(CH_2)_m-CONR^5R^6$, $-CH(Alk)-CONR^5R^6$,

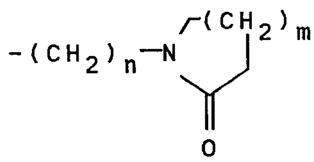,

$-(CH_2)_m$-Aryl, $-(CH_2)_m$-Heteroaryl oder Phenyl bedeuten oder $R^3$ und $R^4$ zusammen mit dem diese bindenden Stickstoffatom einen Heterocyclus bilden;
$R^5$ und $R^6$ unabhängig voneinander Wasserstoff, $(C_1\text{-}C_6)$-Alkyl, $(C_5\text{-}C_7)$-Cycloalkyl, Benzyl, Phenethyl bedeutet;
Alk $(C_1\text{-}C_6)$-Alkyl bedeutet;
n für 2, 3 oder 4 und
m für 1, 2 oder 3 stehen; und
$R^7$ und $R^8$ unabhängig voneinander Wasserstoff, $(C_1\text{-}C_4)$-Alkyl, $(C_1\text{-}C_4)$-Alkoxy, Fluor, Chlor, Brom, Nitro oder Trifluormethyl bedeuten oder eines pharmakologisch annehmbaren Säureadditionssalzes davon zur Behandlung erektiler Dysfunktionen.

**10.** Pharmazeutisches Präparat, dadurch gekennzeichnet, daß es ein Phenyl-1,2,5-oxadiazol-carbonamid-2-oxid der allgemeinen Formel I gemäß einem der Ansprüche 8 oder 9, oder ein pharmakologisch annehmbares Säureadditionssalz davon als Wirkstoff zusammen mit pharmazeutisch annehmbaren Träger- und Zusatzstoffen und gegebenenfalls noch ein oder mehrere andere pharmakologische Wirkstoffe enthält.

Europäisches Patentamt

# EUROPÄISCHER RECHERCHENBERICHT

Nummer der Anmeldung

EP 93 10 8736
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 075 141 (CASSELLA) 30. März 1983 * das ganze Dokument * --- | 1-10 | C07D271/08 A61K31/41 |
| D,A | EP-A-0 054 873 (CASSELLA) 30. Juni 1982 * das ganze Dokument * --- | 1-10 | |
| D,A | EP-A-0 054 872 (CASSELLA) 30. Juni 1982 * das ganze Dokument * --- | 1-10 | |
| A | EP-A-0 038 438 (CASSELLA) 28. Oktober 1981 * das ganze Dokument * --- | 1-10 | |
| A | CH-A-498 137 (J. R. GEIGY) 15. Dezember 1970 * Formel IIa und IIb sowie Spalte 1, Zeile 17-26 * --- | 1-10 | |
| | | | RECHERCHIERTE SACHGEBIETE (Int. Cl.5) |
| A | CH-A-498 856 (J. R. GEIGY) 31. Dezember 1970 * Formel II und Spalte 1, Zeile 21-31 * --- | 1-10 | C07D |
| A | JUSTUS LIEBIGS ANNALEN DER CHEMIE. 1990, WEINHEIM DE Seiten 335 - 338 R. FRUTTERO ET. AL. 'Phenylfuroxancarboxylic Acids and Their Derivatives.' * Verbindungen 12,13 * --- | 1-7 | |
| | -/-- | | |

Der vorliegende Recherchenbericht wurde für alle Patentansprüche erstellt

| Recherchenort | Abschlußdatum der Recherche | Prüfer |
|---|---|---|
| DEN HAAG | 07 OKTOBER 1993 | Bernd Kissler |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 93 10 8736
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | ANNALI DI CHIMICA (ROME) Bd. 58, Nr. 2, 1968, Seiten 200 - 212 M. GRIFANTINI ET. AL. 'Reazioni di .alpha.-ossimmino-derivati di composti carbonilici con basi.' * siehe Verbindungen der Tabelle I auf Seite 202 und exp. Teil * * RN= 18721-31-8, 18721-32-9, 20709-70-0, 20709-71-1 * --- | 1,2,4 | |
| P,A | SCIENCE Bd. 257, 17. Juli 1992, Seiten 401 - 403 S. H. SNYDER ET. AL. 'Nitric Oxide: A Physiological Mediator of Penile Erection.' * das ganze Dokument * --- | 1-10 | |
| L | SCIENTIFIC AMERICAN, SPECIAL ISSUE MEDICINE 1993, REPRINTED FROM THE MAY 1992 ISSUE Mai 1992, NEW YORK Seiten 22 - 29 S. H. SNYDER 'Biological Roles of Nitric Oxide.' * das ganze Dokument * ----- | 1-10 | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| DEN HAAG | 07 OKTOBER 1993 | Bernd Kissler |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument